(19) Europäisches Patentamt

European Patent Office

Office européen des brevets

(11) **EP 1 090 631 A1**

(12) **EUROPEAN PATENT APPLICATION**

(43) Date of publication:
**11.04.2001 Bulletin 2001/15**

(51) Int Cl.⁷: **A61K 7/50**

(21) Application number: **00307849.0**

(22) Date of filing: **11.09.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **09.09.1999 GB 9921205**

(71) Applicant: **Robert McBride Ltd**
**Middleton, Manchester M24 4DP (GB)**

(72) Inventors:
• **Yates, Paul Barrie**
  **Ossett WF5 8HG (GB)**
• **Itoe, Rudolf Duala**
  **Leeds LS2 1EH (GB)**

(74) Representative: **Atkinson, Peter Birch et al**
**MARKS & CLERK,**
**Sussex House,**
**83-85 Mosley Street**
**Manchester M2 3LG (GB)**

(54) **Personal hygiene product**

(57)    A personal hygiene product, one of the uses of which is as a shower gel, which has high viscosity and foaming ability for the consumer yet also has a high clarity. The product includes an anionic surfactant containing an alkyl group, a cross-linked polycarboxylate thickener and a low molecular weight polyol clarifying agent, in the ratios / ranges of 8-11% by weight surfactant, 5-9% by weight clarifier and 1.0-1.4% by weight thickener. A particulate material such as Hakes, beads or encapsulates may be suspended in the product. The encapsulates may contain moisturisers, perfumes, vitamins or oils.

**Description**

The present invention relates to a personal hygiene product, particularly but not exclusively to a fluid cleansing product which has high viscosity and foaming ability.

Recently an increasing number of personal hygiene products, such as body washes, shower and bath gels and hand washes have been available in the marketplace. The fluid products have several advantages over solid soaps and cleansers; they can be dispensed and transported more easily and as they are enclosed within a container there is no problem of soap deterioration, which can affect solid soaps when stored uncovered, as a result of their hygroscopic nature. Fluid products also typically produce more foam more quickly than solid soaps, which is pleasing to the user.

Fluid products may contain suspended particulate material such as flakes, particles such as beads or encapsulates, which not only make them appear more aesthetically pleasing but can also serve an additional purpose since the encapsulates can contain supplementary cleansers, oils or body lotions which are released upon bursting or on contact with water onto the skin of the user. The encapsulates may also be used to provide the composition with a larger amount of, e.g. perfume than would otherwise be possible without affecting the desirable properties of the product, or to include compounds such as vitamin E that would otherwise break down.

Generally personal hygiene products are required to have three properties within an acceptable range. These properties are; thickness, cleaning / foaming ability and clarity. Thickness is especially important when particulate material is added to the product so that the product is viscous enough to prevent the particulate material sinking due to gravity, commonly a thickener such as xanthan gum is used in this respect.

However, an increase in one ingredient (e.g. surfactant, thickener or clarifier), to improve one property of the composition, is detrimental to the other two properties and a compromise must be reached wherein the level of one of the properties is not as high as is desired. Often it is the clarity property that is compromised, e.g. commonly a product composition that has suitable viscosity and cleaning / foaming ability is hazy due to the presence of the thickener. It can be necessary to add pearlisers or other components so that the cleaning product still has an acceptable look to the user. Such pearlisers can obscure the suspended particles, making them more difficult to see.

It is desirable to be able to produce a clear product with sufficient cleaning / foaming ability that is capable of suspending particulate material.

It is an object of the present invention to obviate or mitigate some or all of the disadvantages with prior art compositions.

According to the present invention there is provided a personal hygiene product comprising:

(I) 8-11% by weight of an anionic surfactant containing an alkyl group;
(II) 5-9% by weight of a low molecular weight polyol clarifier; and
(III) 1.0-1.4% by weight of a cross-linked polycarboxylate thickener.

Preferably the anionic surfactant containing an alkyl group comprises 9-11% by weight of the product. The low molecular weight polyol clarifier preferably comprises 5-8% by weight of the product and the cross-linked polycarboxylate thickener preferably comprises 1.1-1.4% by weight of the product.

When the constituents are present in the abovementioned amounts, unexpectedly all of the desired properties (foaming, clarity and viscosity) of the personal hygiene product can be attained without there being any detriment to any one property, as has been observed previously. Personal hygiene products with excellent foaming, clarity and thickness properties may be obtained by providing a composition according to the invention. The personal hygiene products also have excellent particulate suspending ability.

The anionic surfactant containing an alkyl group (hereafter referred to as the anionic surfactant) is preferably in the form of an alkyl sulphate or alkyl ether sulphate.

The alkyl group of the anionic surfactant preferably has at least one $C_{8-22}$ alkyl group. For preference the alkyl group of the anionic surfactant has 8-16 carbon atoms. The anionic surfactant may be a single compound (preferably $C_{12}$) or may comprise a mixture of compounds of different chain length, preferably with $C_{12}$ predominating. For preference the alkyl groups are primary alkyl groups and preferably straight chain.

The cation of the anionic surfactant may for example be an alkali metal (especially sodium), ammonium, triethylammonium or monoethanol ammonium.

Preferred examples of anionic surfactants are chosen from the group comprising monoethanol ammonium lauryl sulphate, sodium lauryl sulphate, sodium lauryl ether sulphate, triethyl ammonium lauryl sulphate, Empicol ESB™ and Texapon MLS™ (supplied by Albright & Wilson and Henkel respectively).

The low molecular weight polyol clarifier may be any low molecular weight polyol as recognised by those skilled in the art. For preference the molecular weight of the low molecular weight polyol is in the range of 60 to 500, more preferably 76 to 200 and most preferably 76 to 92. It is preferred that the low molecular weight polyol has from 2 to 15 hydroxyl groups and most preferably 2 to 5 hydroxyl groups. Preferred examples of which include ethylene glycol, propylene glycol and glycerol.

Generally the low molecular weight polyol is miscible with water

The cross-linked polycarboxylate thickener may be any cross-linked polycarboxylate thickener as recognised by those skilled in the art. Preferably the cross-linked polycarboxylate thickener is derived from an ethylenically unsaturated acid monomer with a free carboxylic acid group.

Examples of suitable carboxylic acids are $\alpha,\beta$-unsaturated carboxylic acids and dicarboxylic acids. Specific example include acrylic acid, methacrylic acid, itaconic acid, iconatic acid, cinnamic acid, crotonic acid, mesaconic acid, carboxymethyl acrylic acid, maleic acid, fumaric acid and the like. Further examples of classes of acids that may be used include vinyl esters of dicarboxylic acids. A still further example of acid that may be used is 1,4-vinyl benzoic acid. Generally the $\alpha,\beta$-unsaturated carboxylic acids (particularly those containing 3 to 8 carbon atoms) are preferred.

The cross-linked polycarboxylate thickener preferably has a degree of cross-linking of less than 2%, more preferably a maximum of 1% (e.g. 0.01% to 1%). One or more of allyl sucrose, allyl pentaerythritol or polymers thereof may be used as a cross-linking agent.

The cross-linked polycarboxylate thickener preferably has a molecular weight of (Mw) of between 500,000 to 3,000,000.

A specific example of a cross-linked polycarboxylate thickener that may be used in accordance with the invention is Carbopol ETD 2020.

Typically the personal hygiene product will be water based.

The personal hygiene product according to the present invention may include any, all or any combination of the following constituents: -

(1) Humectants.
(2) Penetrants.
(3) Chelating agents.
(4) Suspending agents.
(5) Base carriers.
(6) Solvents.
(7) Emulsifying agents.
(8) Neutralising agents.
(9) UV protectors.
(10) Colour stabilisers.
(11) Preservatives.

The personal hygiene product may include suspended particles. Preferred examples of which are selected from the group which includes flakes, solid particles, beads, air bubbles and capsules. It is recognised that capsules may be used to contain desirable additives, which otherwise if free in solution may detrimentally affect the properties of the solution. Generally the additives are oil based. Where capsules are used they may contain one or more of the following: -

(1) Perfume.
(2) Moisturising compositions
(3) Oils, such as mineral oils.
(4) Vegetable oils, such as oil extracted from sunflower, jojoba, grapeseed, mango seed, shea butter, coconut, palm, canola and corn.
(5) Esters.
(6) Silicone oils, especially with a yield above 350.
(7) Essential oils, such as tea tree, ylang ylang, sandalwood and nerloli,
(8) Vitamin compositions, such as compositions including vitamin A and / or E.
(9) Sunscreen, such as oil soluble sunscreen.Colouring agents, such as pearlisers, pigments and oil soluble colours.
(10) Bacteriocides, such as Triclosan™ (available from Ciba-Geigy).

It is preferred that the suspended particles are from 0.3mm to 2.0mm in diameter.

Typically the personal hygiene product is prepared by adding the constituents in the following order: -

1) Water
2) Particles to be suspended
3) Cross-linked carbopol thickener

4) Low molecular weight polyol clarifier

5) Anionic surfactant containing an alkyl group.

Generally the personal hygiene product formulation is carried out at 20°C with continuous stirring.

It is preferred that the personal hygiene product has an absolute viscosity of 16,000 to 30,000 centipoise (when measured on a viscometer (Brookfield DVL+), using spindle 4, set to speed 5, at 20°C). It will be understood by those skilled in the art that the yield figure quoted for a fluid is related to the viscosity of the fluid, wherein the viscosity is measured under two different sets of conditions. The yield figure is recognised as a measure of a fluid's ability to suspend particles. The preferred yield of the personal hygiene product is more than 300.

It is preferred that the personal hygiene product has a foaming ability of between 30 to 40 mm after 30 minutes when measured by the following technique, which includes the steps of: -

(1) Preparation of a 5% w/w solution of the personal hygiene product by weighing 5.0g of the personal hygiene product into a 200ml glass beaker and adding 95cm$^3$ of tap water at about 40°C (avoiding foaming during addition).
(2) Stir the solution gently (avoiding foaming) using a magnetic stirrer until the personal hygiene product is dispersed.
(3) Equilibrate the solution at 40°C.
(4) Pipette 20cm$^3$ of the solution into a 100cm$^3$ measuring cylinder (in which a 1cm height change is associated with a 10cm$^3$ volume change) fitted with a "B" stopper.
(5) With the stopper in place, invert the cylinder to the upside down position and back again, making sure that the liquid falls under gravity and no shaking of the cylinder is carried out.
(6) Repeat step (5) a further 4 times.
(7) Take the reading of the foam height after 10 seconds, 5 minutes and 30 minutes.
(8) Repeat steps (4) to (7) and carry out an average of 4 readings.

It is preferred that the personal hygiene product has a clarity of at least 80 for visible light, more preferably more than 85, even more preferably more than 90 and most preferably more than 95, wherein clarity is defined in the following formula: -

$$Clarity = 100 - (ABS \times 100)$$

where ABS is the absorbance of visible light.

The invention will now be further illustrated with reference to the following non-limiting Examples and the accompanying figures in which:

Figure 1 is a graphical representation of the relationship between viscosity and Carbopol concentration;

Figure 2 is a graphical representation of the relationship between viscosity and surfactant (Empicol ESB and Texapon MLS) concentration;

Figure 3 is a graphical representation of the relationship between viscosity and propylene glycol concentration;

Figure 4 is a graphical representation illustrating the relative amounts of Empicol ESB, Carbopol ETD 2020 and propylene glycol required to obtain a personal hygiene product according to the invention; and

Figure 5 is a graphical representation illustrating the relative amounts of Texapon MLS, Carbopol ETD 2020 and propylene glycol required to obtain a personal hygiene product according to the invention.

**Comparative Example 1-Dependence of Viscosity upon Carbopol Concentration**

Aqueous compositions containing 2% propylene glycol and 10% surfactant (either Empicol ESB or Texapon MLS) with a range of Carbopol ETD 2020 concentrations were made up.

Viscosity was measured using a Brookfield DV1+ viscometer, using spindle 4 set to speed 5, measured at 20°C.

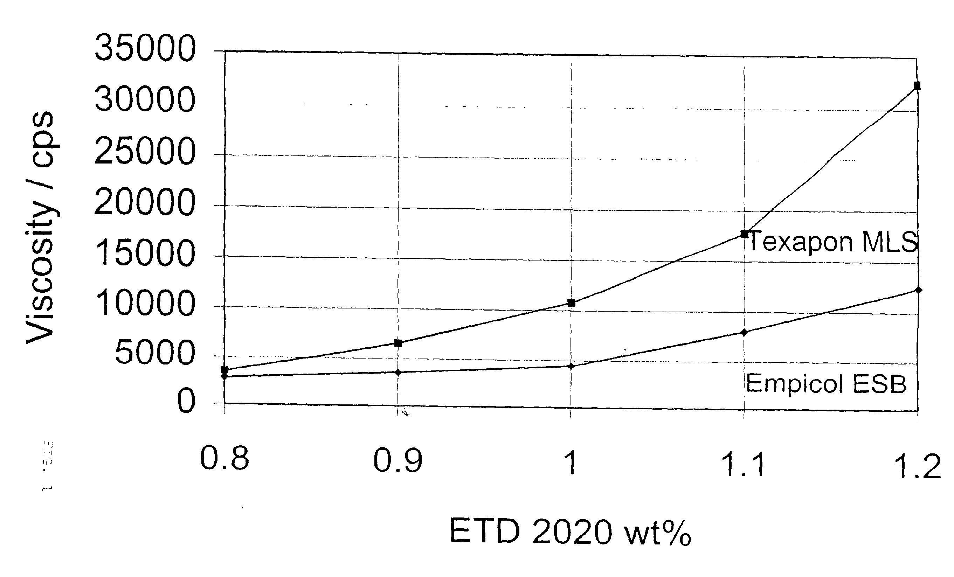

The clarity was judged on a visual scale of 1-6 by 6 assessors. The results of the measurements / assessments are presented in Table 1 and Figure 1.

Table 1

| ETD 2020 (wt%) | Viscosity / cps Empicol ESB (27.5%) | Viscosity / cps Texapon MLS | Clarity ESB | Clarity Texapon MLS |
|---|---|---|---|---|
| 0.8 | 2800 | 3500 | poor | good |
| 0.9 | 3500 | 6540 | poor | poor |
| 1.0 | 4400 | 10800 | unacceptable | poor |
| 1.1 | 8000 | 17800 | unacceptable | unacceptable |
| 1.2 | 12400 | 32400 | unacceptable | unacceptable |

It can be seen that addition of greater quantities of the thickener Carbopol ETD 2020 caused the clarity to become unacceptable.

**Comparative Example 2-Dependence of Viscosity upon Surfactant Concentration**

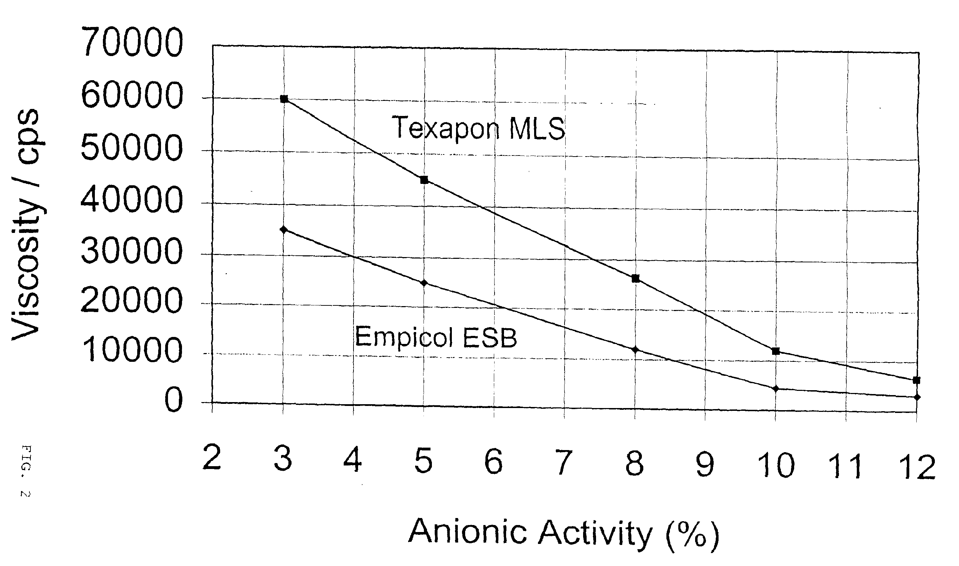

Aqueous compositions containing 2% propylene glycol and 1.0% Carbopol ETD 2020 with a range of surfactant concentrations (either Empicol ESB or Texapon MLS) were made up. The viscosity and clarity of the solutions were measured / assessed as in Example 1. The activity of the surfactant was measured by titration technique using a cationic surfactant and the presence of an organic phase, with a dye indicator to gauge the end point of the reaction. This method is known as two-phase titration.
The results of the tests / assessments are presented in Table 2 and Figure 2.

Table 2

| Anionic Activity (%) | Viscosity / cps Empicol ESB (27.5%) | Viscosity / cps Texapon MLS | Clarity ESB | Clarity Texapon MLS |
|---|---|---|---|---|
| 3 | 35000 | 60000 | good | excellent |
| 5 | 25000 | 45000 | good | good |
| 8 | 12000 | 26400 | poor | good |
| 10 | 4400 | 12000 | unacceptable | poor |
| 12 | 3000 | 6500 | unacceptable | unacceptable |

It can be seen that addition of greater quantities of the surfactant caused both the viscosity and the clarity to become unacceptable

**Comparative Example 3-Dependence of Viscosity upon Clarifier Concentration**

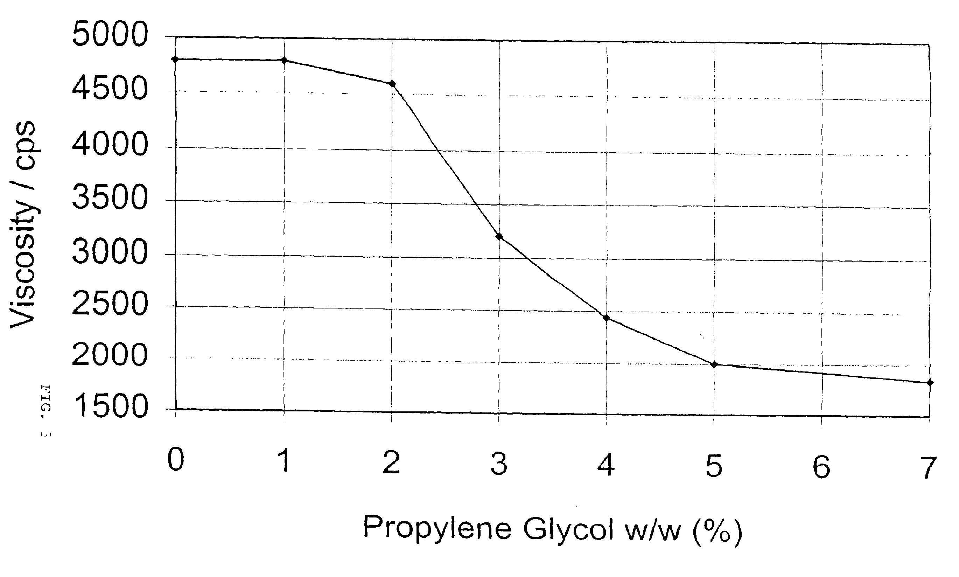

Aqueous compositions containing 10% Empicol ESB and 1.0% Carbopol ETD 2020 with a range of propylene glycol concentrations were made up. The viscosity and clarity of the solutions were measured / assessed as described above, the results of which are presented in Table 3 and Figure 3.

Table 3

| Propylene Glycol (%) | Viscosity / cps | Clarity |
|---|---|---|
| 0 | 4800 | poor |
| 1 | 4800 | poor |
| 2 | 4600 | poor |
| 3 | 3200 | good |
| 4 | 2440 | good |
| 5 | 2000 | v.good |
| 7 | 1840 | excellent |

It can be seen that addition of greater quantities of the propylene glycol caused the clarity to become more acceptable to the detriment of decreasing the viscosity of the solution.

## Comparative Example 4 - Dependence of Foam Height Upon Surfactant Concentration.

Aqueous compositions containing surfactant (either Empicol ESB or Texapon MLS) were made up according to Table 4.

Foam height was measured by, pipetting $20cm^3$ of the solution (at $40°C$) into a $100cm^3$ measuring cylinder (in which a 1 cm height change is associated with a $10cm^3$ volume change) fitted with a "B" stopper.

The cylinder was inverted five times so that the liquid fell under gravity. The foam height was measured after 30minutes. The results are shown in Table 4.

Table 4

| Surfactant Concentration (% w/w) | Foam Height (cm) |
|---|---|
| 6 | 30 |
| 4 | 25 |
| 2 | 23 |

It can be seen that use of surfactant at low concentration (2-6% w/w) produces an unacceptably low foam height.

Comparative Examples 1 to 4 highlight the problem of obtaining a personal hygiene product comprised of a surfactant, a thickener and a clarifier, which has suitable viscosity, foaming ability and clarity.

To achieve an acceptably viscous hygiene product it is recognised that the product must have a viscosity of at least 16,000. As can be seen from Figures 1-3 and Tables 1-4 it is impossible to reach this figure without compromising on the foaming ability or the clarity of the composition. Similarly to reach the desired anionic activity or clarity the viscosity of the product must be compromised.

By Examination of Figures 1-3 and Tables 1-4, it can be seen that a personal hygiene product, as comprising the three ingredients would not be expected to meet the required foaming ability, clarity and viscosity requirements.

## Example 1-Empicol ESB Hygiene Product Formulation

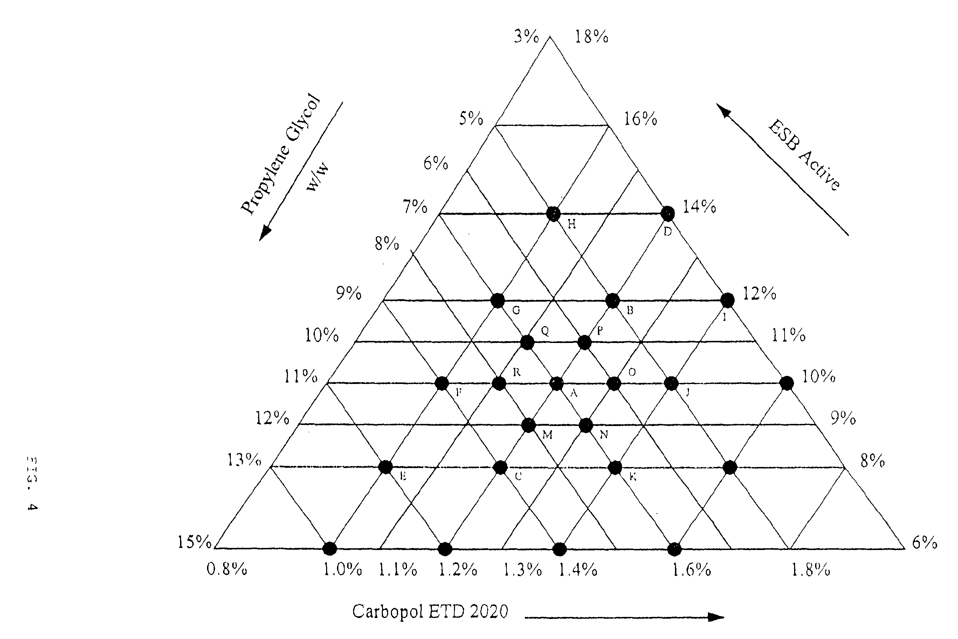

Compositions A to R were prepared containing amounts of Empicol ESB, propylene glycol and Carbopol ETD 2020 as indicated by the points on the triangular graph of Figure 4, in which the axes are; Carbopol ETD 2020 concentration measured in wt%, Empicol ESB activity measured in % and propylene glycol concentration measured in wt%.

Points A, C, J, K, M, N, O and R are within the scope of the invention and are considered to meet the required criteria for the personal hygiene product, i.e. the compositions have a viscosity of between 16,000 to 30,000 centipoise, a yield of over 300, a foaming ability of 30-40mm as described above and a clarity (as defined above) of at least 80 for visible light.

For example, points A, C and K have foam heights of 40 mm, point J has a foam height of 42.5mm and point B has a clarity of 90.9.

Points B, D, E, F, G, H, I, P and Q are not within the scope of the invention and are not considered to meet the required criteria for the personal hygiene product.

## Example 2- Texapon MLS Hygiene Product Formulation

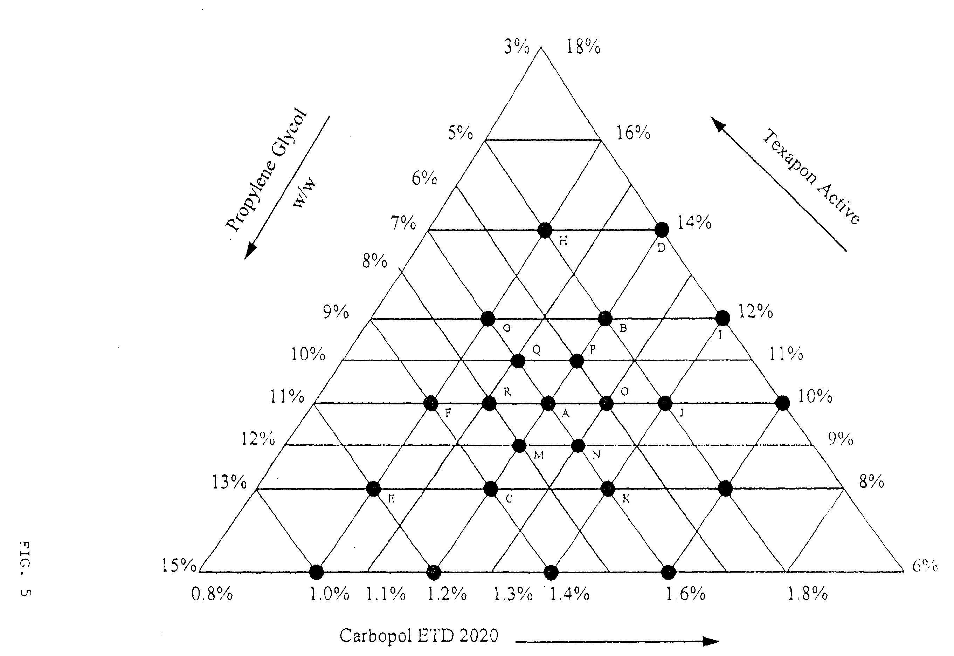

Compositions A to R were prepared containing amounts of Texapon MLS, propylene glycol and Carbopol ETD 2020 as indicated by the points on the triangular graph of Figure 5, in which the axes are; Carbopol ETD 2020 concentration measured in wt%, Empicol ESB activity measured in % and propylene glycol concentration measured in wt%.

Points A, C, E, J, K, M, N, O, P, Q and R are within the scope of the invention and are considered to meet the required criteria for the personal hygiene product, i.e. the compositions have a viscosity of between 16,000 to 30,000 centipoise, a yield of over 300, a foaming ability of 30-40 mm as described above and a clarity (as defined above) of at least 80 for visible light.

For example point E has a clarity if 97.6.

Points B, D, F, G, H, and I are not within the scope of the invention and are not considered to meet the required criteria for the personal hygiene product.

For example point D has a clarity of only 79.1.

Other ingredients for a suitable product (whether for the compositions exemplified in Example 1 or Example 2) are shown in Table 5. The functions of these ingredients are also included.

Table 5

| Raw Material | Wt% | Function |
|---|---|---|
| Water | To 100 | Base Carrier |
| SLES / Texapon MLS | 8.0 - 11.0** | Primary Surfactant |
| Carbopol ETD 2020 | 1.0-1.4 | Suspending agent |
| Propylene Glycol | 5.0-9.0 | Humectant / Solvent |
| Glycerine | 2.0 | Humectant |
| Polysorbate 40 (Croda) | 1.5 | Penetrant |
| Triethanolamine | qs | Neutralising Agent |
| Disodium Deterate | 0.1 | Chelating Agent |
| Uvasorb | qs | UV Protector / Colour Stabiliser |
| Euxyl K100 | qs | Preservative |

** added as 31-33% w/w aqueous solutions.

### Example 3- Clarity of Personal Hygiene Formulation

The absorbance of the compositions in Table 6 were measured on a UV Spectrophotometer (Philips PU 800 Series) set at 600nm with continuous scanning.

The clarity is calculated using the formula below: -

$$Clarity = 100 - (ABS \times 100)$$

Table 6

| Propylene Glycol ((w/w)%) | Carbopol ETD 2020 (%) | Surfactant Active (%) | ABS | Clarity |
|---|---|---|---|---|
| 0 | 0 | 0 | 0.002 | 99.8 |
| 9 | 1.2 | 8* | 0.075 | 92.5 |
| 5 | 1.4 | 10 | 0.180 | 81.0 |
| 11 | 1.0 | 8 | 0.319 | 68.1 |

All of the samples were made up to 100% with water. * Denotes Texapon MLS used as the surfactant, in the other samples Empicol ESB was used. The final sample is a comparative example, it illustrates how the clarity falls to an unacceptable level when the thickener concentration (Carbopol ETD 2020) and the clarity agent (propylene glycol) fall outside the acceptable ranges of the invention.

### Claims

1. A personal hygiene product comprising:

   (I) 8-11% by weight of an anionic surfactant containing an alkyl group;
   (II) 5-9% by weight of a low molecular weight polyol clarifier; and
   (III) 1.0-1.4% by weight of a cross-linked polycarboxylate thickener.

2. A personal hygiene product according to claim 1, wherein the amount of surfactant is from 9-11wt%.

**3.** A personal hygiene product according to claim 1 or 2, wherein the amount of clarifier is from 5.0-8.0wt%.

**4.** A personal hygiene product according to claim 1, 2 or 3, wherein the amount of thickener is from 1.1-1.4wt%.

**5.** A personal hygiene product according to any of claims 1 to 4, wherein the anionic surfactant containing an alkyl group is an alkyl sulphate or an alkyl ether sulphate.

**6.** A personal hygiene product according to claim 5, wherein the alkyl group of the anionic surfactant has a $C_{8-22}$ alkyl group.

**7.** A personal hygiene product according to claim 6, wherein the alkyl group of the anionic surfactant has a $C_{8-16}$ alkyl group.

**8.** A personal hygiene product according to any of claims 5 to 7, wherein the cation for the surfactant is an alkali metal (especially sodium), ammonium, triethyl ammonium or monoethanol ammonium.

**9.** A personal hygiene product according to any of claims 5 to 8, wherein the surfactant is selected from the group comprising monoethanol ammonium lauryl sulphate, sodium lauryl sulphate, sodium lauryl ether sulphate, triethyl ammonium lauryl sulphate, Empicol ESB™ and Texapon MLS™.

**10.** A personal hygiene product according to any of claims 1 to 9, wherein the molecular weight of the low molecular weight polyol is in the range of 60 to 500.

**11.** A personal hygiene product according to claim 10, wherein the molecular weight of the low molecular weight polyol is in the range of 76 to 200.

**12.** A personal hygiene product according to claim 11, wherein the molecular weight of the low molecular weight polyol is in the range of 76 to 92.

**13.** A personal hygiene product according to any of claims 1 to 9, wherein the low molecular weight polyol has from 2 to 15 hydroxyl groups.

**14.** A personal hygiene product according to claim 13, wherein the low molecular weight polyol has from 2 to 5 hydroxyl groups.

**15.** A personal hygiene product according to claim 13 or 14, where the low molecular weight polyol is selected from the group comprising ethylene glycol, propylene glycol and glycerol.

**16.** A personal hygiene product according to any of claims 1 to 15, wherein the thickener is Carbopol ETD 2020.

**17.** A personal hygiene product according to any of claims 1 to 16, which includes any combination of humectants, penetrants, chelating agents, suspending agents, base carriers, solvents, emulsifying agents, neutralising agents, UV protectors, colour stabilisers, preservatives.

**18.** A personal hygiene product according to any of claims 1 to 17, wherein the product includes suspended particles.

**19.** A personal hygiene product according to claim 18, wherein the suspended particles are in the form of flakes, solid particles, beads, air bubbles and capsules.

**20.** A personal hygiene product according to claim 19, wherein the capsules contain moisturising compositions, oils, such as mineral oils, vegetable oils, such as oil extracted from sunflower, jojoba, grapeseed, mango seed, shea butter, coconut, palm, canola or corn, esters, silicone oils with a yield above 350, essential oils, such as tea tree, ylang ylang, sandalwood or nerloli, vitamin compositions, such as compositions including vitamin A or E, sunscreen, such as oil soluble sunscreen, colouring agents, such as pearlisers, pigments or oil soluble colours or bacteriocides, such as Triclosan™.

**21.** A personal hygiene product according to claim 18, 19 or 20, wherein the particles are from 0.3mm to 2.0mm in diameter.

22. A personal hygiene product according to any of claims 1 to 21, wherein the product has a viscosity of 16,000 to 30,000 centipoise, when measured on a viscometer (Brookfield DVL+), using spindle 4, set to speed 5, at 20°C.

23. A personal hygiene product according to any of claims 1 to 22, wherein the product has a yield of more than 300.

24. A personal hygiene product according to any of claims 1 to 23, wherein the product has a foaming ability of between 30 to 40mm when after 30 minutes $20cm^3$ of a 5% w/w aqueous solution of the product is inverted 5 times in a $100cm^3$ beaker.

25. A personal hygiene product according to any of claims 1 to 24, wherein the product has a clarity of at least 80 for visible light.

26. A personal hygiene product according to claim 25, wherein the product has a clarity of at least 85 for visible light.

27. A personal hygiene product according to claim 26, wherein the product has a clarity of at least 90 for visible light.

28. A personal hygiene product according to claim 27, wherein the product has a clarity of at least 95 for visible light.

EP 1 090 631 A1

FIG. 2

FIG. 3

FIG. 4

FIG. 5

**EP 1 090 631 A1**

**European Patent Office**

**EUROPEAN SEARCH REPORT**

Application Number

EP 00 30 7849

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | EP 0 950 400 A (KAO CORP) 20 October 1999 (1999-10-20) * page 3, line 50 - page 4, line 14; claim 1; example 4; table 4 * --- | | A61K7/50 |
| A | US 5 750 122 A (COX BRUCE RUSSELL ET AL) 12 May 1998 (1998-05-12) * column 6, line 20 - line 36; example 21 * ----- | | |

TECHNICAL FIELDS SEARCHED (Int.Cl.7)

A61K

The present search report has been drawn up for all claims

| Place of search | Date of completion of the search | Examiner |
|---|---|---|
| THE HAGUE | 4 January 2001 | Stienon, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 00 30 7849

This annex lists the patent family members relating to the patent documents cited in the above-mentioned European search report.
The members are as contained in the European Patent Office EDP file on
The European Patent Office is in no way liable for these particulars which are merely given for the purpose of information.

04-01-2001

| Patent document cited in search report | | Publication date | Patent family member(s) | | Publication date |
|---|---|---|---|---|---|
| EP 0950400 | A | 20-10-1999 | JP | 11292728 A | 26-10-1999 |
| | | | JP | 11310797 A | 09-11-1999 |
| US 5750122 | A | 12-05-1998 | BR | 9707008 A | 20-07-1999 |
| | | | CN | 1213297 A | 07-04-1999 |
| | | | EP | 0874619 A | 04-11-1998 |
| | | | JP | 11502872 T | 09-03-1999 |
| | | | WO | 9725973 A | 24-07-1997 |

EPO FORM P0459

For more details about this annex : see Official Journal of the European Patent Office, No. 12/82